# EUROPÄISCHE PATENTSCHRIFT

(11) **EP 0 657 423 B1**
(45) Veröffentlichungstag und Bekanntmachung des Hinweises auf die Patenterteilung: **30.12.1998**
(21) Anmeldenummer: 94118931.8
(22) Anmeldetag: 01.12.1994
(51) Int. Cl.: C07C 311/58, C07C 335/42, C07D 295/135

(54) **Aminosubstituierte Benzolsulfonylharnstoffe und -thioharnstoffe, Verfahren zu ihrer Herstellung und ihre Verwendung als Pharmazeutika**
Aminosubstituted benzoylsulfonylurea and thiourea derivatives, process for their preparation and their use as pharmaceuticals
Urées et thiourées benzolsulfonylées amino substituées, procédé pour leur préparation et leur utilisation comme produits pharmaceutiques

(30) Priorität: 07.12.1993 DE 4341655
(43) Veröffentlichungstag der Anmeldung: 14.06.1995
(73) Patentinhaber: HOECHST AKTIENGESELLSCHAFT, 65929 Frankfurt am Main (DE)
(72) Erfinder: Englert, Heinrich, Dr., D-65719 Hofheim (DE); Mania, Dieter, Dr., D-61462 Königstein (DE); Hartung, Jens, Dr., D-97204 Höchberg (DE); Gögelein, Heinz, Dr., D-60259 Frankfurt (DE); Kaiser, Joachim, Dr., D-60431 Frankfurt (DE)

(56) Entgegenhaltungen:
- DE-A- 1 518 879
- CARDIOVASCULAR DRUGS AND THERAPY, Bd.7, 1. August 1993 Seite 507-513 L. H. OPIE 'Modulation of ischemia by regulation of the ATP-sensitive potassium channel'

## Beschreibung

Die Erfindung betrifft substituierte Benzolsulfonylharnstoffe und -thioharnstoffe I 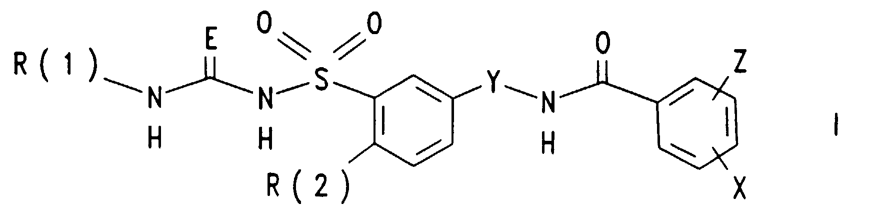 worin bedeuten:
- R(1): Wasserstoff, (C₁-C₆)-Alkyl, (CH₂)ᵣ-CₚF₂ₚ₊₁, (C₃-C₆)-Cycloalkyl, (C₁-C₂)-Alkyl-(C₃-C₅)-Cycloalkyl oder (C₂-C₆)-Alkenyl;
r null, 1, 2, 3, 4 oder 5;
p 1, 2, 3, 4, 5 oder 6;
- R(2): NR(3)R(4);
R(3) und R(4) gemeinsam
   eine (CH₂)₂₋₇-Kette, in der bei einer Kettenlänge von 4 bis 7 eine der CH₂-Gruppen durch Sauerstoff, Schwefel oder NR(5) ersetzt sein kann, wobei zwischen dem N-Atom des NR(3)R(4) und dem Sauerstoff, Schwefel oder NR(5) mindestens eine CH₂-Gruppe stehen muß;
   oder
R(3), R(4), R(5) unabhängig voneinander
   Wasserstoff, (C₁-C₆)-Alkyl, (CH₂)ᵣ-CₚF₂ₚ₊₁, (C₃-C₆)-Cycloalkyl, (C₁-C₂)-Alkyl-(C₃-C₅)-Cycloalkyl oder (C₂-C₆)-Alkenyl;
   r Null, 1, 2, 3, 4 oder 5;
   p 1, 2, 3, 4, 5 oder 6;
- E: Sauerstoff oder Schwefel;
- Y: eine Kohlenwasserstoffkette der Formel -[CR(6)₂]ₙ-; R(6) Wasserstoff oder (C₁-C₂)-Alkyl;
n 1, 2, 3 oder 4;
- X: Wasserstoff, F, Cl, Br, 1 oder (C₁-C₆)-Alkyl;
- Z: F, Cl, Br, l, NO₂, (C₁-C₄)-Alkoxy oder (C₁-C₄)-Alkyl;
und ihre physiologisch unbedenklichen Salze.

Der Begriff Alkyl beschreibt, sofern nicht anders angegeben, geradkettige, oder verzweigte, gesättigte Kohlenwasserstoffreste. Der Cycloalkylrest kann zusätzlich Alkylsubstituenten tragen.

Als Halogensubstituenten sind die Elemente Fluor, Chlor, Brom und lod einsetzbar. Die Kohlenstoffatome der Alkylseitenkette Y können asymmetrisch substituiert sein. Zur Erfindung gehören dabei Verbindungen des einen oder des anderen Enantiomers sowie einer racemischen Mischung oder Mischungen der Antipoden in unterschiedlichen Verhältnissen. Weiterhin können Verbindungen mit zwei bis vier Chiralitätszentren in der Alkylseitenkette Y auftreten. In diesem Fall gehören sowohl die einzelnen Antipoden für sich, als auch eine Mischung der Enantiomere oder Diastereomere in unterschiedlichen Verhältnissen, sowie die dazugehörigen Mesoverbindungen oder Mischungen aus Mesoverbindungen, den Enantiomeren oder Diastereomeren zur Erfindung.

Ähnliche Sulfonylharnstoffe sind aus der deutschen Offenlegungsschrift 2 413 514 und der deutschen Patentschrift 1 518 874 sowie der DE-A-1 518 879 bekannt. Die DE-OS 2 413 514 beschreibt ausschließlich Blutzuckerkonditionierende Stoffe mit p-Substitution in der zentralen Phenylgruppe. Hinweise auf Aminosubstituenten finden sich nicht.

In den Patent-Veröffentlichungen wird die blutzuckersenkende Wirkung der Sulfonylharnstoffe beschrieben. Als Prototyp solcher blutzuckersenkenden Sulfonylharnstoffe gilt das Glibenclamid, das als Mittel zur Behandlung des Diabetes mellitus therapeutisch verwendet wird und in der Wissenschaft als ein vielbeachtetes Werkzeug zur Erforschung sogenannter ATP sensitiver Kaliumkanäle dient. Neben seiner blutzuckersenkenden Wirkung besitzt das Glibenclamid noch andere Wirkungen, die bislang therapeutisch noch nicht eingesetzt werden können, die aber allesamt auf Blockade eben dieser ATP-sensitiven Kalium-Kanäle zurückgeführt werden. Dazu gehört insbesondere eine antifibrillatorische Wirkung am Herzen (siehe zum Beispiel L. H. Opie, Modulation of Ischemia by Regulation of the ATP-Sensitive Potassium Channel, Cardiovascular Drugs and Therapy, Vol. 7, Seite 507-513 (1993)). Bei der Behandlung des Kammerflimmerns oder seiner Vorstufen wäre jedoch eine gleichzeitige Blutzuckersenkung unerwünscht oder gar gefährlich, da sie den Zustand des Patienten weiter verschlechtern kann.

Aufgabe der vorliegenden Erfindung war es daher, Verbindungen zu synthetisieren, die eine gleich gute Herzwirkung wie Glibenclamid aufweisen, aber den Blutzucker in herzwirksamen Dosen oder Konzentrationen nicht oder deutlich geringer beeinflussen als Glibenclamid.

Als Versuchstiere zum Nachweis solcher Wirkungen eignen sich zum Beispiel Mäuse, Ratten, Meerschweinchen, Kaninchen, Hunde, Affen oder Schweine.

Die Verbindungen I dienen als Arzneimittelwirkstoffe in der Human- und Veterinärmedizin. Ferner können sie als Zwischenprodukte zur Herstellung weiterer Arzneimittelwirkstoffe verwendet werden.

Bevorzugt sind die Verbindungen I, in denen bedeuten:
- R(1): Wasserstoff, (C₁-C₄)-Alkyl, CₚF₂ₚ₊₁, (C₃-C₅)-Cycloalkyl, CH₂-(C₃-C₅)-Cycloalkyl oder (C₃-C₄)-Alkenyl;
p 1, 2 oder 3;
- R(2): NR(3)R(4);
R(3) und R(4) gemeinsam
   eine (CH₂)₂₋₇-Kette, in der bei einer Kettenlänge von 4 bis 7 eine der CH₂-Gruppen durch Sauerstoff, Schwefel oder NR(5) ersetzt sein kann, wobei zwischen dem N-Atom des NR(3)R(4) und dem Sauerstoff, Schwefel oder NR(5) mindestens eine CH₂-Gruppe stehen muß;
   oder
R(3), R(4), R(5) unabhängig voneinander
   Wasserstoff, (C₁-C₆)-Alkyl, (CH₂)ᵣ-CₚF_{2p + 1}, (C₃-C₆)-Cycloalkyl, (C₁-C₂)-Alkyl-(C₃-C₅)-Cycloalkyl oder (C₂-C₆)-Alkenyl;
   r Null, 1, 2, 3, 4 oder 5;
   p 1, 2, 3, 4, 5 oder 6;
- E: Sauerstoff oder Schwefel;
- Y: eine Kohlenwasserstoffkette der Formel -[CR(6)₂]ₙ-;
R(6) Wasserstoff oder (C₁-C₂)-Alkyl;
n 1, 2, 3 oder 4;
- X: Wasserstoff, F, Cl oder (C₁-C₄)-Alkyl;
- Z: Cl, F, (C₁-C₄)-Alkyl oder (C₁-C₄)-Alkoxy. Insbesondere sind die Verbindungen I bevorzugt, in denen bedeuten: R(1) Wasserstoff, (C₁-C₄)-Alkyl, (C₃-C₄)-Cycloalkyl oder (C₃-C₄)-Alkenyl; R(2) NR(3)R(4),
R(3) und R(4) gemeinsam
   eine (CH₂)₄₋₆ Kette, in der eine CH₂-Gruppe durch Sauerstoff, Schwefel oder N-R(5) ersetzt sein kann, wobei zwischen dem N-Atom des NR(3)R(4) und dem Sauerstoff, Schwefel oder NR(5) mindestens eine CH₂-Gruppe stehen muß,
   oder
R(3) und R(4) unabhängig voneinander
   CH₃, C₂H₅, n-Propyl, iso-Propyl, cyclo-Propyl;
R(5) Wasserstoff, CH₃ oder C₂H₅;
- E: Sauerstoff oder Schwefel;
- Y: eine Kohlenwasserstoffkette der Formel -[CR(6)₂]ₙ-; R(6) Wasserstoff oder Methyl;
n 2 oder 3;
- X: Wasserstoff, Cl, F oder (C₁-C₃)-Alkyl;
- Z: F, Cl oder (C₁-C₃)-Alkoxy.

Die Verbindungen I der vorliegenden Erfindung sind wertvolle Arzneimittel zur Behandlung von Herzrhythmusstörungen unterschiedlichster Genese und zur Verhinderung des arrhythmisch bedingten, plötzlichen Herztodes und können daher als Antiarrhythmika Verwendung finden. Beispiele arrhythmischer Störungen des Herzens sind supraventrikuläre Rhythmusstörungen, wie etwa Vorhof-Tachykardien, Vorhofflattern oder paroxysmale supraventrikuläre Rhythmusstörungen, oder ventrikuläre Rhythmusstörungen, wie ventrikuläre Extrasystolen, insbesondere aber lebensbedrohende ventrikuläre Tachykardien oder das besonders gefährliche Kammerflimmern. Sie eignen sich insbesondere für solche Fälle, in denen Arrhythmien Folge einer Verengung eines Koronargefäßes sind, wie sie beispielsweise bei Angina Pectoris oder während eines akuten Herzinfarkts oder als chronische Folge eines Herzinfarkts auftreten. Sie sind daher insbesondere bei Postinfarktpatienten zur Verhinderung des plötzlichen Herztodes geeignet. Weitere Krankheitsbilder, bei denen derartige Rhythmusstörungen und/oder der plötzliche, arrhythmisch bedingte Herztod eine Rolle spielen sind beispielsweise die Herzinsuffizienz oder die Herzhypertrophie als Folge eines chronisch erhöhten Blutdruckes.

Darüberhinaus können die Verbindungen I eine verminderte Kontraktilität des Herzens positiv beeinflussen. Hierbei kann es sich um ein krankheitsbedingtes Nachlassen der Herzkontraktilität handeln, beispielsweise bei Herzinsuffizienz, aber auch um akute Fälle wie Herzversagen bei Schockeinwirkungen. Ebenso kann bei einer Herztransplantation das Herz nach erfolgter Operation seine Leistungsfähigkeit rascher und zuverlässiger wieder aufnehmen. Gleiches gilt für Operationen am Herz, die eine vorübergehende Stillegung der Herzaktivität durch kardioplegische Lösungen erforderlich machen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können.

Die Erfindung betrifft weiterhin ein Verfahren zur Herstellung der Verbindungen I dadurch gekennzeichnet, daß man
(a) aromatische Sulfonamide der Formel II oder deren Salze der Formel III 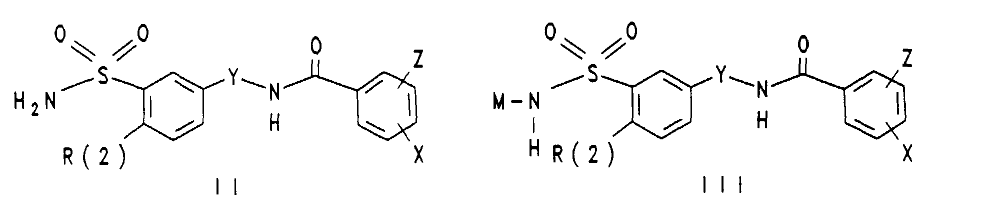 mit einem R(1)-substituierten Isocyanat der Formel IV

   R(1) - N = C = O IV

   zu substituierten Benzolsulfonylharnstoffen I a umsetzt.
   Als Kationen M in den Salzen der Formel III kommen Alkali- und Erdalkaliionen in Betracht. Äquivalent zu den R(1)-substituierten Isocyanaten IV kann man R(1)-substituierte Carbamidsäureester, R(1)-substituierte Carbamidsäurehalogenide oder R(1)-substituierte Harnstoffe einsetzen.
(b) Unsubstituierte Benzolsulfonylharnstoffe I a [R(1) = H] 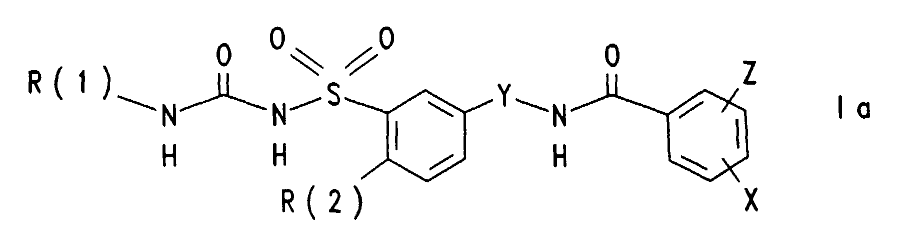 kann man durch Umsetzungen aromatischer Benzolsulfonamide der Formel II oder deren Salze III mit Trialkylsilylisocyanat oder Siliciumtetraisocyanat und Hydrolyse der primären siliciumsubstituierten Benzolsulfonylharnstoffe herstellen. Weiterhin ist es möglich, Benzolsulfonamide II oder deren Salze III durch Umsetzung mit Halogencyanen und Hydrolyse der primär entstehenden N-Cyanosulfonamide mit Mineralsäuren bei Temperaturen zwischen 0 und 100°C darzustellen.
(c) Benzolsulfonylharnstoffe I a 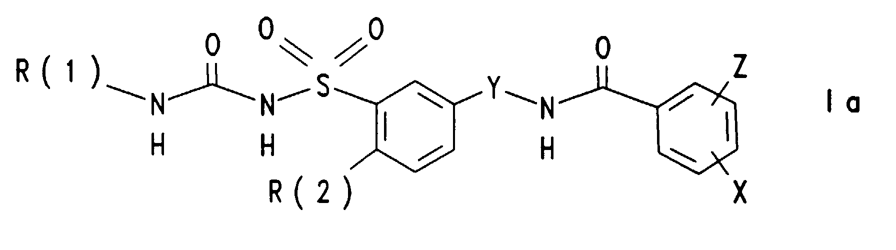 lassen sich aus aromatischen Benzolsulfonamiden II oder deren Salzen III und R(1)-substituierten Trichloracetamiden der Formel V 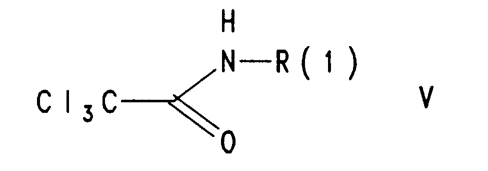 in Gegenwart einer Base in einem inerten Lösungsmittel nach Synthesis 1987, 734 bis 735 bei Temperaturen von 25 bis 150°C darstellen.
   Als Basen eignen sich zum Beispiel Alkalimetall- oder Erdalkalimetallhydroxide, -hydride, -amide oder auch -alkoholate, wie Natriumhydroxid, Kaliumhydroxid, Calciumhydroxyd, Natriumhydrid, Kaliumhydrid, Calciumhydrid, Natriumamid, Kaliumamid, Natriummethylat, Natriumethanolat, Kaliummethylat oder Kaliumethanolat. Als inerte Lösungsmittel eignen sich Ether wie Tetrahydrofuran, Dioxan, Ethylenglykoldimethylether (Diglyme), Nitrile wie Acetonitril, Ester wie Ethylacetat, Carbonsäureamide wie Dimethylformamid (DMF) oder N-Methylpyrrolidon (NMP), Sulfoxide wie DMSO, Sulfone wie Sulfolan, Kohlenwasserstoffe wie Benzol, Toluol, Xylole. Weiterhin eignen sich auch Gemische dieser Lösungsmittel untereinander.
(d) Benzolsulfonylthioharnstoffe I b 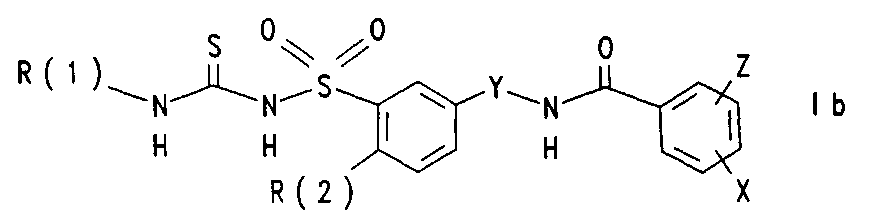 werden aus Benzolsulfonamiden II sowie deren Salze III und R(1)-substituierten Thioisocyanaten IV

   R(1) - N = C = S IV

   dargestellt.
(e) Substituierte Benzolsulfonylharnstoffe der Formel I a können durch Umwandlungsreaktionen von Benzolsulfonylthioharnstoffen der Struktur I b dargestellt werden. Der Ersatz des Schwefelatoms durch ein Sauerstoffatom in den entsprechend substituierten Benzolsulfonylthioharnstoffen I b kann man beispielsweise mit Hilfe von Oxidationsmitteln wie Wasserstoffperoxid, Natriumperoxid oder Salpetersäure erreichen. Thioharnstoffe können auch durch Behandlung mit Phosgen oder Phosphorpentachlorid entschwefelt werden. Als Zwischenverbindungen werden Chlorameisensäureamidine bzw. Carbodiimide erhalten, die zum Beispiel durch Verseifung bzw. Anlagerung von Wasser in die entsprechenden substituierten Benzolsulfonylharnstoffe I a überführt werden. Isothioharnstoffe verhalten sich bei der Entschwefelung wie Thioharnstoffe und können demzufolge ebenso als Ausgangsstoffe für diese Reaktionen dienen.
(f) Benzolsulfonylharnstoffe I a lassen sich durch Umsetzungen von Aminen der Formel R(1)-NH₂ mit Benzolsulfonylisocyanaten der Formel VII 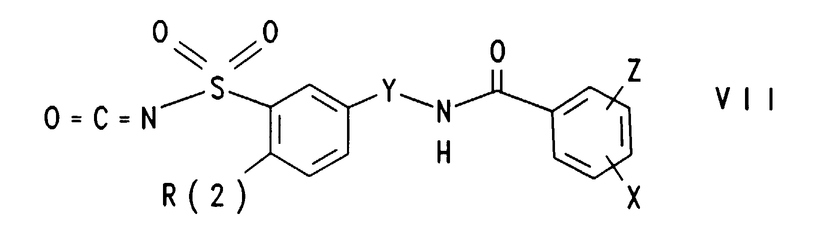 herstellen. Ebenso können Amine R(1)-NH₂ mit
   Benzolsulfonylcarbamidsäureestern, -carbamidsäurehalogeniden oder Benzolsulfonylharnstoffen I a [mit R(1) = H] zu den Verbindungen I a umgesetzt werden.
(g) Benzolsulfonylthioharnstoffe I b lassen sich durch Umsetzungen von Aminen der Formel R(1)-NH₂ mit Benzolsulfonylisothiocyanaten der Formel VIII 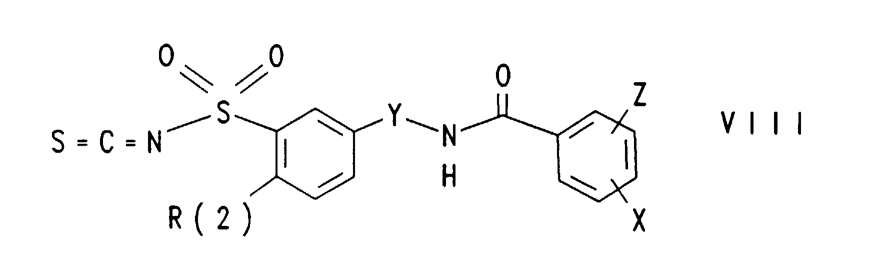 herstellen.
   Ebenso können Amine R(1)-NH₂ mit
   Benzolsulfonylcarbamidsäurethioestern, -carbamidsäurethiohalogeniden zu den Verbindungen I b umgesetzt werden.

Die Verbindungen I sowie deren physiologisch unbedenkliche Salze sind wertvolle Therapeutika, die sich nicht nur als Antiarrhythmika, sondern auch als Prophylaxe bei Störungen des kardiovaskulären Systems, Herzinsuffizienz, Herztransplantation oder cerebralen Gefäßerkrankungen an Menschen oder Säugetieren (zum Beispiel Affen, Hunde, Mäuse, Ratten, Kaninchen, Meerschweinchen und Katzen) eignen.

Unter physiologisch unbedenklichen Salzen der Verbindungen I versteht man nach Remmington's Pharmaceutical Science, 17. Auflage, 1985, Seiten 14 bis 18 Verbindungen der Formel X, 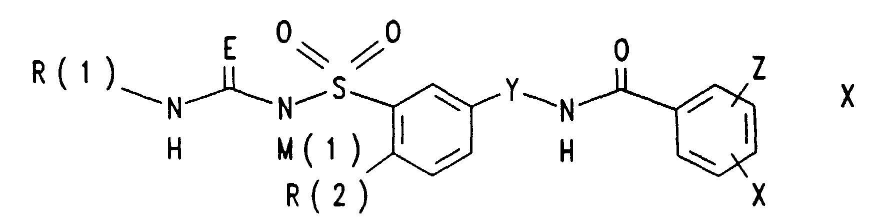 die sich aus nicht toxischen organischen und anorganischen Basen und substituierten Benzolsulfonylharnstoffen I darstellen lassen.

Bevorzugt werden hierbei Salze, in denen M(1) in der Formel X Natrium-, Kalium, Rubidium, Calcium, Magnesium sind, sowie die Säureadditionsprodukte basischer Aminosäuren, wie zum Beispiel Lysin oder Arginin.

Die Ausgangsverbindungen für die erwähnten Syntheseverfahren der Benzolsulfonylharnstoffe I werden nach an sich bekannten Methoden hergestellt, wie sie in der Literatur (zum Beispiel in den Standardwerken wie Houben-Weyl, Methoden der Organischen Chemie, Georg Thieme Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Ausgangsstoffe können gewünschtenfalls auch in situ gebildet werden, derart, daß man sie aus dem Reaktionsgemisch nicht isoliert, sondern sofort weiter umsetzt.

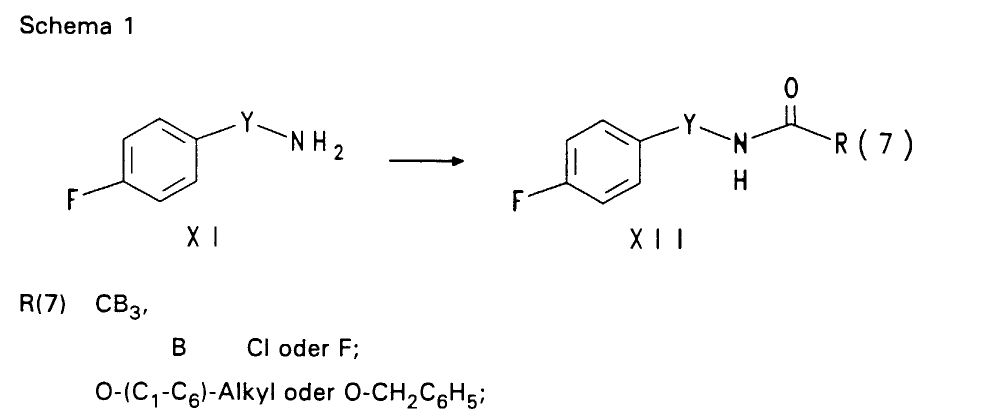

So kann man Fluor-substituierte Phenylalkylamine nach Schema 1 acylieren. Zur Acylierung von Aminogruppen eignen sich zweckmäßig die Alkylester, Halogenide (z. B. Chloride oder Bromide) oder Anhydride von Carbonsäuren der Formel 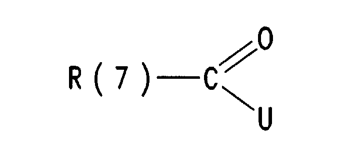 wobei R(7) in Schema 1 definiert ist und U eine Fluchtgruppe wie Halogenid, (C₁-C₄)-Alkoxy, Trihalogenacetat, (C₁-C₄)-Carboxylat ist.

Die nach Schema 1 acylierten Amine XII können in bekannter Weise nach 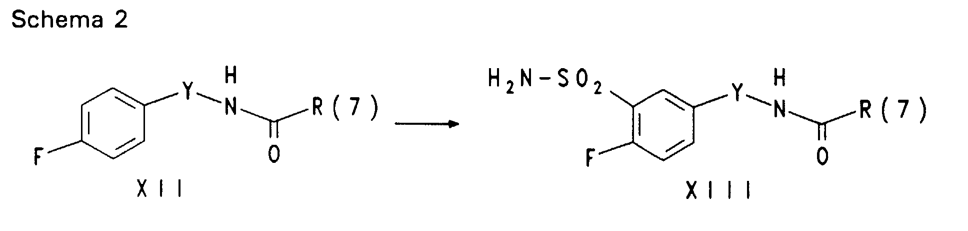 in die Sulfonamide XIII überführt werden. Die Solfonamide XIII werden nach an sich bekannten Methoden hergestellt und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Synthesen können gewünschtenfalls in einem, zwei oder mehreren Schritten vollzogen werden. Insbesondere sind Verfahren bevorzugt, in denen das acylierte Amin XII durch elektrophile Reagenzien in An- oder Abwesenheit von inerten Lösungsmitteln bei Temperaturen von -10 bis 120°C, vorzugsweise von 0 bis 100°C in aromatische Sulfonsäuren sowie deren Derivate wie zum Beispiel Sulfonsäurehalogenide überführt werden. Beispielsweise können Sulfonierungen mit Schwefelsäure oder Oleum, Halogensulfonierungen mit Halogensulfonsäuren, Reaktionen mit Sulfurylhalogeniden in Gegenwart von wasserfreien Metallhalogeniden oder Thionylhalogeniden in Gegenwart von wasserfreien Metallhalogeniden mit anschließenden, in bekannter Weise durchgeführten Oxidationen zu aromatischen Sulfonsäurechloriden, durchgeführt werden. Sind Sulfonsäuren die primären Reaktionsprodukte, so können diese entweder direkt oder durch Behandlung mit tertiären Aminen, wie zum Beispiel Pyridin oder Trialkylaminen oder mit Alkali- oder Erdalkalihydroxiden oder Reagenzien, die diese basischen Verbindungen in situ bilden, in bekannter Weise durch Säurehalogenide wie zum Beispiel Phosphortrihalogenide, Phosphorpentahalogenide, Phosphoroxychloride, Thionylhalogenide, Oxalylhalogenide, in Sulfonsäurehalogenide überführt werden. Die Überführung der Sulfonsäurederivate in Sulfonamide erfolgt in literaturbekannter Weise, vorzugsweise werden Sulfonsäurechloride in inerten Lösungsmitteln bei Temperaturen von 0 bis 100°C mit wäßrigem Ammoniak umgesetzt.

Die Sulfonamide XIII werden nach Schema 3 mit Aminen der Formel HNR(3)R(4) bei Temperaturen von 25 bis 160°C in An- oder Abwesenheit inerter Lösungsmittel zu den aminosubstituierten Sulfonamiden XIV umgesetzt.

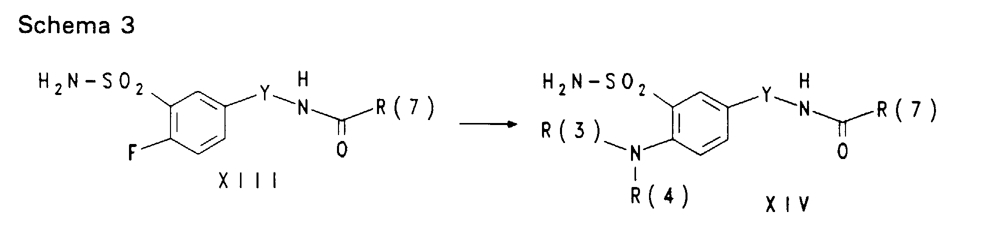

Die Acyl-Schutzgruppe des Amins XIV läßt sich mit Säuren oder Basen abspalten. Durch Spaltung mit wäßrigen Säuren oder Säuren in inerten Lösungsmitteln kann das zugehörige Säureadditionssalz entstehen. Für diese Umsetzung kommen zum Beispiel Schwefelsäure, Halogenwasserstoffsäuren, wie Chlorwasserstoffsäure oder Bromwasserstoffsäure, Phosphorsäuren wie Orthophosphorsäure, Polyphosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, zum Beispiel Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Phenylessigsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Napthalin-mono- und disulfonsäuren, Laurylschwefelsäure in Betracht.

Die basische Spaltung des acylierten Amins der Formel XIV kann in wäßrigen oder inerten Lösungsmitteln erfolgen. Als Basen eignen sich zum Beispiel Alkalimetall- oder Erdalkalimetallhydroxide, oder auch -alkoholate, wie Natriumhydroxid, Kaliumhydroxid, Calciumhydroxyd, Natriummethylat, Natriumethanolat, Kaliummethylat oder Kaliumethanolat, sowie Reduktionsmittel wie NaBH₄ und andere Borane bzw. Boranate.

Aus den so hergestellten sulfonamidsubstituierten Aminen oder deren Säureadditionsverbindungen werden, wie oben erwähnt, die aromatischen Benzolsulfonamide der Formel II hergestellt. Je nach der Natur der Glieder R(1), R(2), R(3), R(4), R(5), R(6), E, X, Y und Z wird in einzelnen Fällen das eine oder andere der genannten Verfahren für die Herstellung der Verbindungen I ungeeignet sein oder zumindest Vorkehrungen zum Schutz aktiver Gruppen notwendig machen. Derartige Fälle können vom Fachmann unschwer erkannt werden, und es sollte in solchen Fällen keine Schwierigkeiten bereiten, einen anderen Syntheseweg erfolgreich anzuwenden.

Die Verbindungen I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Isomere, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen eignen sich zum Beispiel optisch aktive Säuren, wie die R- bzw. R,R- und S- bzw. S,S-Formen von Weinsäure, Dibenzoylweinsäure, Diacetylweinsäure, Camphersulfonsäure, Mandelsäure, Äpfelsäure oder Milchsäure. Carbinole können ferner mit Hilfe chiraler Acylierungsreagenzien, zum Beispiel R- oder S-α-Methylbenzylisocyanat amidiert und dann getrennt werden. Die verschiedenen Formen der Diastereomeren können in an sich bekannter Weise, zum Beispiel durch fraktionierte Kristallisation, getrennt und die Enantiomeren der Formel I können in an sich bekannter Weise aus den Diastereomeren in Freiheit gesetzt werden. Enantiomerentrennungen gelingen ferner durch Chromatographie an optischaktiven Trägermaterialien.

Die erfindungsgemäßen Verbindungen I und ihre physiologisch unbedenklichen Salze können zur Herstellung pharmazeutischer Zubereitungen verwendet werden. Hierbei können sie zusammen mit mindestens einem festen, flüssigen Träger oder Hilfsstoff allein oder in Kombination mit anderen Herz-Kreislaufaktiven Arzneimitteln, wie etwa Calcium-Antagonisten, NO-Donatoren oder ACE-Hemmern in eine geeignete Dosierungsform gebracht werden. Diese Zubereitungen können als Arzneimittel in der Human- oder Veterinärmedizin verwendet werden. Als Trägerstoffe kommen organische oder anorganische Substanzen in Frage, die sich für die enterale (zum Beispiel orale), parenterale, wie zum Beispiel die intravenöse Applikation, oder topische Anwendungen eignen und mit den neuen Verbindungen nicht reagieren, beispielsweise Wasser, pflanzliche Öle, Benzylalkohole, Polyethylenglykole, Glycerintricacetat, Gelatine, Kohlenhydrate, wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Lösungen, vorzugsweise ölige oder wäßrige Lösungen, ferner Suspensionen, Emulsionen oder Implantate, für die topische Anwendung Salben, Cremes, Pasten, Lotionen, Gele, Sprays, Schäume, Aerosole, Lösungen (zum Beispiel in Alkoholen, wie Ethanol oder Isopropanol, 1,2-Propandiol oder deren Gemische untereinander oder mit Wasser) oder Puder. Die neuen Verbindungen können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht, die Stabilisierungs- und/oder Netzmittel, Emulgatoren, Salze und/oder Hilfsstoffe wie Gleit-, Konservierungs-, Stabilisierungs- und/oder Netzmittel, Salze zur Beeinflussung des osmotischen Druckes, Puffersubstanzen, Farb- und Geschmacks- und/ oder Aromastoffe enthalten. Sie können, falls erwünscht, auch einen oder mehrere weitere Wirkstoffe enthalten, zum Beispiel ein oder mehrere Vitamine.

Die Dosierungen, die zur Behandlung von Herzrhythmusstörungen mit den Verbindungen I notwendig sind, hängen davon ab, ob akut oder prophylaktisch therapiert wird. Normalerweise kommt man mit einem Dosisbereich von mindestens etwa 0.1 mg, vorzugsweise etwa 1 mg, insbesondere mindestens etwa 10 mg, bis höchstens 100 mg, vorzugsweise höchstens 50 mg pro kg und Tag aus, wenn Prophylaxe betrieben wird. Ganz besonders bevorzugt ist ein Dosisbereich von 1 bis 10 mg pro kg und Tag. Die Dosis kann dabei als orale oder parenterale Einzeldosis in bis zu vier Einzeldosen aufgeteilt werden. Werden akute Fälle von Herzrhythmusstörungen behandelt, beispielsweise auf einer Intensivstation, kann die parenterale Verabreichung vorteilhaft sein. Ein bevorzugter Dosisbereich in kritischen Situationen kann dann 10 bis 100 mg betragen und beispielsweise als intravenöse Dauerinfusion verabreicht werden.

Erfindungsgemäß können außer den in den Ausführungsbeispielen beschriebenen Verbindungen die in der folgenden Tabelle zusammengestellten Verbindungen I erhalten werden:
(1) 2-Methoxy-5-fluor-N-{5-[-1-sulfonylamino-N-(methylaminothiocarbonyl)-2-(4-morpholino)-phenyl]-ethyl}-benzamid,
(2) 2-Methoxy-5-chlor-N-{5-[-1-sulfonylamino-N-(methylaminocarbonyl)-2-(4-morpholino)-phenyl]-ethyl}-benzamid,
(3) 2-Methoxy-5-chlor-N-{5-[-1-sulfonylamino-N-(methylaminocarbonyl)-2-(4-morpholino)-phenyl]-(3-propyl)}-benzamid,
(4) 2-Methoxy-5-chlor-N-{5-[-1-sulfonylamino-N-(methylaminocarbonyl)-2-(4-thiomorpholino)-phenyl]-ethyl}-benzamid,
(5) 2-Methoxy-5-fluor-N-{5-[-1-sulfonylamino-N-(methylaminothiocarbonyl)-2-(4-thiomorpholino)-phenyl]-ethyl}-benzamid,
(6) 2-Methoxy-5-chlor-N-{5-[-1-sulfonylamino-N-(methylaminocarbonyl)-2-(4-thiomorpholino)-phenyl]-ethyl}-benzamid,
(7) 2-Methoxy-5-fluor-N-{5-[-1-sulfonylamino-N-(methylaminocarbonyl)-2-(4-N-methylpiperazyl)-phenyl]-ethyl}-benzamid,
(8) 2-Methoxy-5-chlor-N-{5-[-1-sulfonylamino-N-(methylaminothiocarbonyl)-2-(4-N-methylpiperazyl)-phenyl]-ethyl}-benzamid,
(9) 2-Methoxy-5-chlor-N-{5-[-1-sulfonylamino-N-(methylaminocarbonyl)-2-(N,N-dimethylamino)-phenyl]-ethyl}-benzamid,
(10) 2-Methoxy-5-fluor-N-{5-[-1-sulfonylamino-N-(methylaminothiocarbonyl)-2(N,N-dimethylamino)-phenyl]-ethyl}-benzamid,
(11) 2-Methoxy-5-chlor-N-{5-[-1-sulfonylamino-N-(methylaminocarbonyl)-2-(N,N-diethylamino)-phenyl]-ethyl}-benzamid,
(12) 2-Methoxy-5-chlor-N-{5-[-1-sulfonylamino-N-(methylaminocarbonyl)-2-(1-pyrrolidyl)-phenyl]-ethyl}-benzamid.

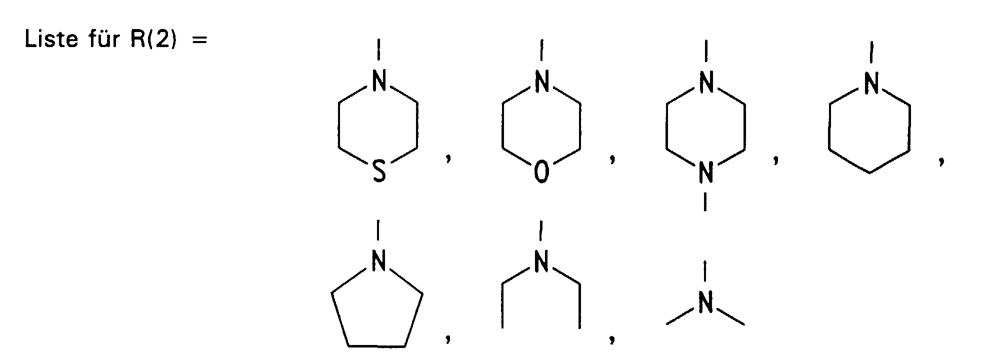

### Beispiel 1:

2-Methoxy-5-chlor-N-{5-[1-sulfonylamino-N-(methylaminothiocarbonyl)-2-(4-morpholino)-phenyl]-ethyl}-benzamid. 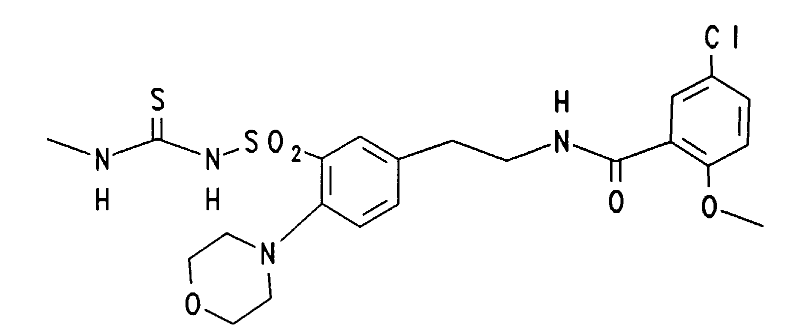 0.45 g (1.0 mmol) 2-Methoxy-5-chlor-N-[5-(1-sulfonylamino-2-(4-morpholino)phenyl)-ethyl]-benzamid werden unter Argon in 5 ml trockenem DMF gelöst und bei 0°C mit 42 mg Natriumhydrid (60 %ige Dispersion in Weißöl) versetzt. Das Kältebad wird entfernt und die Reaktionsmischung 30 Minuten bei Raumtemperatur nachgerührt. In die Lösung des Natriumsulfonamids trägt man 0.10 g Methylisothiocyanat ein und rührt 5 Stunden bei Raumtemperatur sowie 1 Stunde bei 70°C nach. Nach dem Abkühlen gießt man die Reaktionsmischung auf 50 ml 0.5N Salzsäure. Das ausgefallene Produkt wird abgesaugt und getrocknet. Ausbeute: 96 %, Schmp.: 195 bis 196°C.

### Herstellung der Ausgangsverbindung:

1.39 g (10.0 mmol) 4-Fluor-β-phenylethylamin werden in 40 ml Pyridin gelöst, mit einer Spatelspitze Dimethylaminopyridin versetzt und mit einer Lösung von 2.15 g (10.5 mmol) 2-Methoxy-5-chlorbenzoylchlorid versetzt. Die Reaktionsmischung wird auf kalte verdünnte Salzsäure gegossen, das ausgefallene Produkt abgesaugt und getrocknet. Man erhält 4-Fluor-β-phenylethyl-(2-methoxy-5-chlorbenzamid) als farblose Kristalle vom Schmelzpunkt 85°C. Das so erhaltene Benzamid wird in kalte Chlorsulfonsäure eingetragen. Nach vollständiger Umsetzung gießt man die Reaktionsmischung auf Eis, saugt ab (Schmelzpunkt des Sulfonsäurechlorids: 118°C) und löst den Niederschlag in Aceton. Diese Lösung wird mit überschüssigem, konzentriertem, wäßrigem Ammoniak versetzt und nach Abklingen der exothermen Reaktion auf ein Drittel des ursprünglichen Volumens eingeengt. Das 2-Methoxy-5-chlor-N-[5-(1-sulfonylamino-2-fluorphenyl)-ethyl]-benzamid bildet farblose Kristalle, die bei 203°C schmelzen. Dreistündiges Erhitzen von 2-Methoxy-5-chlor-N-[5-(1-sulfonylamino-2-fluorphenyl)-ethyl]-benzamid in überschüssigem Morpholin unter Rückfluß liefert 2-Methoxy-5-chlor-N-[5-(1-sulfonylamino-2-(4-morpholino)-phenyl)-ethyl]-benzamid, das durch Säulenchromatographie (Kieselgel 60, Heptan/Ethylacetat Gradient von 2:1 nach 4:1) isoliert wird. Das Produkt schmilzt bei 236°C.

### Beispiel 2:

2-Methoxy-5-chlor-N-{5-[1-sulfonylamino-N-(methylaminothiocarbonyl)-2-(1-piperidyl)-phenyl]-ethyl}-benzamid. 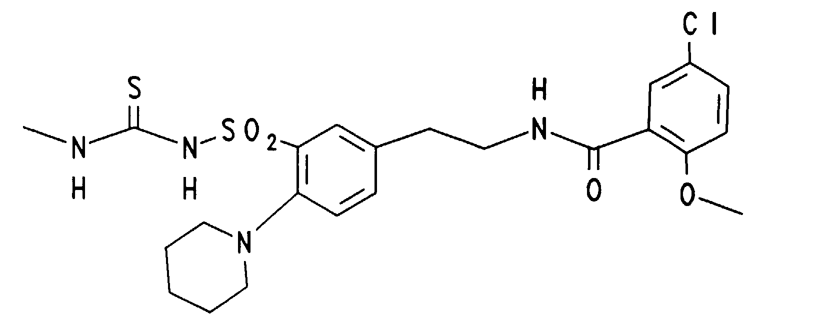 0.45 g (1.0 mmol) 2-Methoxy-4-chlor-N-[5-(1-sulfonylamino-2-(1-piperidyl)phenyl)-ethyl]-benzamid werden unter Argon in 5 ml trockenem DMF gelöst und bei 0°C mit 42 mg Natriumhydrid (60 %ige Dispersion in Weißöl) versetzt. Das Kältebad wird entfernt und die Reaktionsmischung 30 Minuten bei Raumtemperatur nachgerührt. Zur Lösung des Natriumsulfonamids fügt man 0.10 g Methylisothiocyanat und rührt 5 Stunden bei Raumtemperatur sowie 1 Stunde bei 70°C nach. Nach dem Abkühlen auf 50 ml 0.5N Salzsäure gegossen. Das ausgefallene Produkt wird abgesaugt und getrocknet. Ausbeute: 95 %, Schmp.: 90°C.

### Herstellung der Ausgangsverbindung:

Eine Lösung von 2-Methoxy-5-chlor-N-[5-(1-sulfonylamino-2-fluorphenyl)-ethyl]benzamid in überschüssigem Piperidin wird 4 Stunden unter Rückfluß erhitzt. Nach Abdestillieren des Lösungsmittels im Vakuum läßt sich 2-Methoxy-5-chlor-N-[5-(1-sulfonylamino-2-(1-piperidyl)-phenyl)-ethyl]-benzamid säulenchromatographisch (Kieselgel 60, Heptan/Ethylacetat Gradient von 2:1 nach 4:1) als farblose Kristalle vom Schmelzpunkt 225°C isolieren.

### Pharmakologische Daten:

Mit den folgenden Modellen können die therapeutischen Eigenschaften der Verbindungen I nachgewiesen werden:
(1) Aktionspotentialdauer am Papillarmuskel des Meerschweinchens:
(a) Einleitung
ATP-Mangelzustände, wie sie während einer Ischämie in der Herzmuskelzelle beobachtet werden, führen zu einer Verkürzung der Aktionspotentialdauer. Sie gelten als eine der Ursachen für sogenannte Reentryarrhythmien, die den plötzlichen Herztod verursachen können. Die Öffnung von ATP-sensitiven K-Kanälen durch das Absinken von ATP gilt hierfür als ursächlich.
(b) Methode
Zur Messung des Aktionspotentials wird eine Standard-Mikroelektroden-Technik eingesetzt. Hierfür werden Meerschweinchen beiderlei Geschlechts durch Schlag auf den Kopf getötet, die Herzen entnommen, die Papillarmuskeln herausgetrennt und in einem Organbad aufgehängt. Das Organbad wird mit Ringerlösung (0.9 % NaCl, 0.048 % KCl, 0.024 % CaCl₂, 0.02 % NaHCO₃, und 0.1 % Glucose) durchspült und mit einer Mischung aus 95 % Sauerstoff und 5 % Kohlendioxid bei einer Temperatur von 36°C begast. Der Muskel wird über eine Elektrode mit Rechteck-Impulsen von 1 V und 1 ms Dauer und einer Frequenz von 2 Hz angeregt. Das Aktionspotential wird durch eine intrazellulär eingestochene Glas-Mikroelektrode, die mit 3 mMol KCl-Lösung gefüllt ist, abgeleitet und registriert. Die zu prüfenden Substanzen wurden der Ringerlösung in einer Konzentration von 2.2•10⁻⁵ Mol pro Liter zugesetzt. Das Aktionspotential wird mit einem Amplifier von Hugo Sachs verstärkt auf einem Oszilloskop dargestellt. Die Dauer des Aktionspotentials wird bei einem Repolarisierungsgrad von 95 % (APD₉₅) bestimmt. Aktionspotentialverkürzungen werden entweder durch Zugabe einer 1 µM starken Lösung des Kaliumkanalöffners Rilmakalim (Hoe 234) [W. Linz, E. Klaus, U. Albus, R.H.A. Becker, D. Mania, H. C. Englert, B. A. Schölkens Arzneimittelforschung/Drug Research, Band 42 (II), 1992, S. 1180 bis 1185] oder durch Zugabe von 2-Desoxyglucose (DEO) hervorgerufen. Durch 2-Desoxyglucose werden in der experimentellen Physiologie ATP-Mangelzustände durch Blockade des Glucosestoffwechsels hervorgerufen. Der aktionspotential-verkürzende Effekt dieser Substanzen wurde durch die gleichzeitige Gabe der Testsubstanzen verhindert oder veringert. Testsubstanzen wurden als Stammlösungen in Propandiol der Badlösung zugesetzt. Die angegebenen Werte beziehen sich auf Messungen 30 Minuten nach der Zugabe. Als Kontrolle gilt die APD₉₅ in Gegenwart von DEO oder Rilmakalim und in Abwesenheit der Testsubstanz.
(c) Resultate:
Folgende Werte wurden gemessen:

| Messung | APD₉₅-DEO^{a)} [ms] | APD₉₅-Rilmakalim^{a)} [ms] |
|---|---|---|
| Kontrolle | < 40 | < 40 |
| Beispiel 1 | 110 (172) n = 1 | 121 ± 14 (150 ± 9) n = 3 |
| Beispiel 2 | 105 ± 11 (141 ± 4) n = 3 | 153 ± 15 (158 ± 12) n = 3 |

| | | |
|---|---|---|
| ^{a)} den Meßwerten aus n-Versuchen sind die korrespondierenden Leerwerte in Klammern nachgestellt. Die Leerwerte sind die APD₉₅-Werte zu Versuchsbeginn ohne DEO, Rilmakalim und Testsubstanz in der Ringerlösung. | | |

(2) Membranpotential an isolierten β-Zellen:
(a) Einleitung
Der Wirkmechanismus der blutzuckersenkenden Sulfonylharnstoffe ist in groben Zügen aufgeklärt. Zielorgan sind die β-Zellen des Pankreas wo es zu einer Mehrausschüttung des blutzuckersenkenden Hormons Insulin kommt. Die Freisetzung von Insulin wird über das Zellmembranpotential gesteuert. Glibenclamid bewirkt eine Depolarisation der Zellmembran, was über einen vermehrten Einstrom von Calciumionen die Insulinfreisetzung fördert. Das Ausmaß dieser Depolarisation der Zellmembran ΔU wurde an RlNm5F Zellen, einer Pankreastumorzellinie, für einige der erfindungsgemäßen Verbindungen bestimmt. Die Wirkstärke einer Verbindung in diesem Modell sagt das Ausmaß des blutzuckersenkenden Potentials dieser Verbindung voraus.
(b) Methode
Zellkultur von RlNm5F Zellen
RlNm5F Zellen wurden in RPMI 1640 Kulturmedium (Flow), dem 11 mmol Glukose, 10 % (vol/vol) fötales Kälberserum, 2 mmol Glutamin und 50 µg/ml Gentamycin zugesetzt wurde, bei 37°C kultiviert. Für die Untersuchungen wurden die Zellen durch Inkubation (ca. 3 Minuten) in einem Ca²⁺-freien Medium, das 0.25 % Trypsin enthielt, isoliert und auf Eis aufbewahrt. Meßmethode
Isolierte RINm5F Zellen wurden in eine Plexiglaskammer auf einem inversen Mikroskop, das mit Differential-Interferenz-Kontrast Optik ausgerüstet ist, gebracht. Unter optischer Kontrolle (400-fache Vergrößerung) wurde eine feuerpolierte Mikropipette mit Öffnungsdurchmessser von etwa 1 µm mit Hilfe eines Mikromanipulators auf die Zelle aufgesetzt. Durch Anlegen eines leichten Unterdruckes in der Patch-Pipette wurde zunächst eine hohe elektrische Abdichtung zwischen Glas und Zellmembran hergestellt und anschließend durch Erhöhung des Unterdruckes der Membranfleck unter der Meßpipette aufgerissen. In dieser Ganzzellenkonfiguration wurde mit Hilfe eines Patch-Clamp Verstärkers (L/M EPC 7) das Zellpotential registriert und durch Anlegen einer Spannungsrampe der Ganzzellenstrom gemessen.
Lösungen: Die Patch-Pipette war mit KCl-Lösung gefüllt (in mmol): 140 KCl, 10 NaCl, 1.1 MgCl₂, 0.5 EGTA, 1 Mg-ATP, 10 HEPES, pH = 7.2, und im Bad befand sich NaCl-Lösung (in mmol): 140 NaCl, 4.7 KCl, 1.1 MgCl₂, 2 CaCl₂, 10 HEPES, pH = 7.4. Von den Testsubstanzen wurden Stocklösungen (Konzentration 100 mmol) in Dimethylsulfoxid (DMSO) und entsprechende Verdünnungen in NaCl-Lösung hergestellt. DMSO alleine hatte keinen Effekt auf das Zellpotential. Um das Zellpotential unter Kontrollbedingungen zu stabilisieren, wurde der Öffner für ATP-sensitive K⁺ Kanäle Diazoxid (100 µmol) bei allen Versuchen in die Badlösung zugegeben. Alle Experimente wurden bei 34 ± 1 °C durchgeführt.
(c) Ergebnisse (Die Konzentrationen der erfindungsgemäßen Verbindungen betragen in den Versuchen 10⁻⁶ Mol pro Liter)

| Messung | ΔU (mv)^{a}⁾ |
|---|---|
| Beispiel 1 | 14 (-76) n = 7 |
| Beispiel 2 | 19 (-76) n = 3 |

| | |
|---|---|
| ^{a)} den Meßwerten aus n-Versuchen sind die korrespondierenden Leerwerte in Klammern nachgestellt. Die Leerwerte sind die Zellpotentiale unter Diazoxid-Gabe. | |

## Patentansprüche

1. Substituierte Benzolsulfonylharnstoffe und -thioharnstoffe I 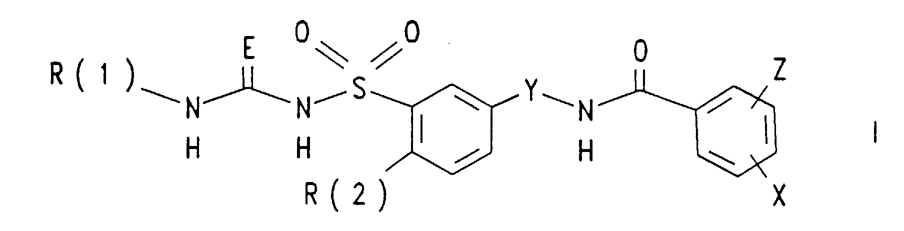 worin bedeuten:
R(1) Wasserstoff, (C₁-C₆)-Alkyl, (CH₂)ᵣ-CₚF₂ₚ₊₁, (C₃-C₆)-Cycloalkyl, (C₁-C₂)-Alkyl-(C₃-C₅)-Cycloalkyl oder (C₂-C₆)-Alkenyl;
r Null, 1, 2, 3, 4 oder 5;
p 1, 2, 3, 4, 5 oder 6;
R(2) NR(3)R(4),
R(3) und R(4) gemeinsam
eine (CH₂)₂₋₇-Kette, in der bei einer Kettenlänge von 4 bis 7 eine der CH₂-Gruppen durch Sauerstoff, Schwefel oder NR(5) ersetzt sein kann, wobei zwischen dem N-Atom des NR(3)R(4) und dem Sauerstoff, Schwefel oder NR(5) mindestens eine CH₂-Gruppe stehen muß;
oder
R(3), R(4), R(5) unabhängig voneinander
Wasserstoff, (C₁-C₆)-Alkyl, (CH₂)ᵣ-CₚF₂ₚ₊₁, (C₃-C₆)-Cycloalkyl, (C₁-C₂)-Alkyl-(C₃-C₅)-Cycloalkyl oder (C₂-C₆)-Alkenyl;
r Null, 1, 2, 3, 4 oder 5;
p 1, 2, 3, 4, 5 oder 6;
E Sauerstoff oder Schwefel;
Y eine Kohlenwasserstoffkette der Formel -[CR(6)₂]ₙ-;
R(6) Wasserstoff oder (C₁-C₂)-Alkyl;
n 1, 2, 3 oder 4;
X Wasserstoff, F, Cl, Br, 1 oder (C₁-C₆)-Alkyl;
Z F, Cl, Br, l, NO₂, (C₁-C₄)-Alkoxy oder (C₁-C₄)-Alkyl;
und ihre physiologisch unbedenklichen Salze.

2. Verbindung der Formel I nach Anspruch 1, dadurch gekennzeichnet, daß darin bedeuten:
R(1) Wasserstoff, (C₁-C₄)-Alkyl, CₚF₂ₚ₊₁, (C₃-C₅)-Cycloalkyl, CH₂-(C₃-C₅)-Cycloalkyl oder (C₃-C₄)-Alkenyl;
p 1, 2 oder 3;
R(2) NR(3)R(4),
R(3) und R(4) gemeinsam
eine (CH₂)₂₋₇-Kette, in der bei einer Kettenlänge von 4 bis 7 eine der CH₂-Gruppen durch Sauerstoff, Schwefel oder NR(5) ersetzt sein kann, wobei zwischen dem N-Atom des NR(3)R(4) und dem Sauerstoff, Schwefel oder NR(5) mindestens eine CH₂-Gruppe stehen muß;
oder
R(3), R(4), R(5) unabhängig voneinander
Wasserstoff, (C₁-C₆)-Alkyl, (CH₂)ᵣ-CₚF₂ₚ₊₁, (C₃-C₆)-Cycloalkyl, (C₁-C₂)-Alkyl-(C₃-C₅)-Cycloalkyl oder (C₂-C₆)-Alkenyl;
r Null, 1, 2, 3, 4 oder 5;
p 1, 2, 3, 4, 5 oder 6;
E Sauerstoff oder Schwefel;
Y eine Kohlenwasserstoffkette der Formel -[CR(6)₂]ₙ-;
R(6) Wasserstoff oder (C₁-C₂)-Alkyl;
n 1, 2, 3 oder 4;
X Wasserstoff, F, Cl oder (C₁-C₄)-Alkyl;
Z Cl, F, (C₁-C₄)-Alkyl oder (C₁-C₄)-Alkoxy.

3. Verbindung der Formel I nach Anspruch 1 und/oder 2, dadurch gekennzeichnet, daß darin bedeuten:
R(1) Wasserstoff, (C₁-C₄)-Alkyl, (C₃-C₄)-Cycloalkyl oder (C₃-C₄)-Alkenyl;
R(2) NR(3)R(4),
R(3) und R(4) gemeinsam
eine (CH₂)₄₋₆ Kette, in der eine CH₂-Gruppe durch Sauerstoff, Schwefel oder N-R(5) ersetzt sein kann, wobei zwischen dem N-Atom des NR(3)R(4) und dem Sauerstoff, Schwefel oder NR(5) mindestens eine CH₂-Gruppe stehen muß,
oder
R(3) und R(4) unabhängig voneinander
CH₃, C₂H₅, n-Propyl, Iso-Propyl oder Cyclo-Propyl;
R(5) Wasserstoff, CH₃ oder C₂H₅;
E Sauerstoff oder Schwefel;
Y eine Kohlenwasserstoffkette der Formel -[CR(6)₂]ₙ-;
R(6) Wasserstoff oder Methyl;
n 2 oder 3;
X Wasserstoff, Cl, F oder (C₁-C₃)-Alkyl;
Z F, Cl oder (C₁-C₃)-Alkoxy.

4. Verfahren zur Herstellung einer Verbindung I nach einem oder mehreren der Ansprüche 1 bis 3, dadurch gekennzeichnet, daß man
(a) aromatische Sulfonamide der Formel II oder deren Salze der Formel III 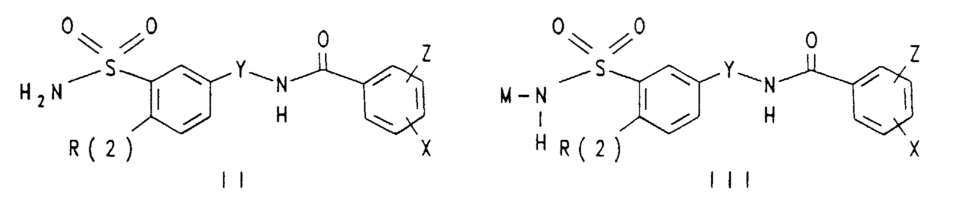 mit R(1)-substituierten Isocyanaten der Formel IV
R(1) - N = C = O IV
zu substituierten Benzolsulfonylharnstoffen I a umsetzt,
oder
(b) einen unsubstituierten Benzolsulfonylharnstoff I a [R(1) = H] 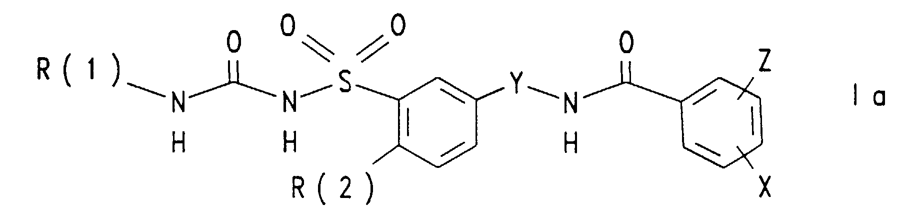 durch Umsetzungen eines aromatischen Benzolsulfonamids der Formel II oder von dessen Salz III mit Trialkylsilylisocyanat oder
Siliciumtetraisocyanat und Hydrolyse des primären siliciumsubstituierten Benzolsulfonylharnstoffs herstellt,
oder
(c) einen Benzolsulfonylharnstoff I a 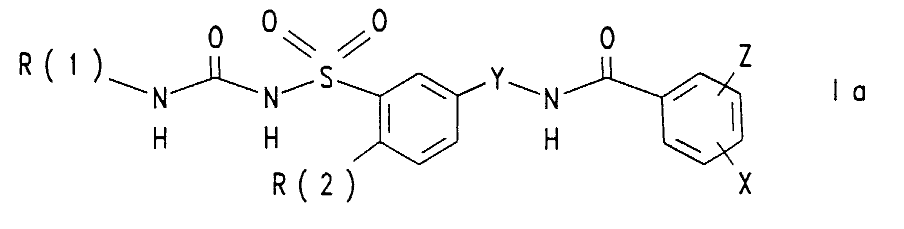 aus einem aromatischen Benzolsulfonamid II oder dessen Salz III und R(1)-substituierten Trichloracetamiden der Formel V 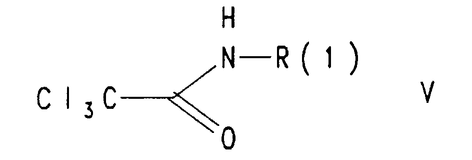 in Gegenwart einer Base darstellt,
oder
(d) einen Benzolsulfonylthioharnstoff I b 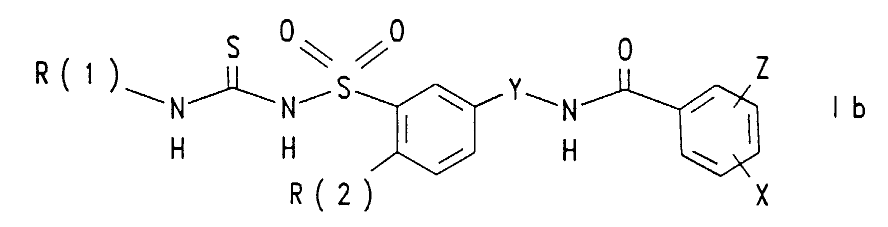 aus einem Benzolsulfonamid II oder dessen Salz III und R(1)-substituierten Thioisocyanaten IV
R(1) - N = C = S IV
darstellt,
oder
(e) einen Benzolsulfonylharnstoff I a durch Umsetzung eines Amins der Formel R(1)-NH₂ mit einem Benzolsulfonylisocyanat der Formel VII 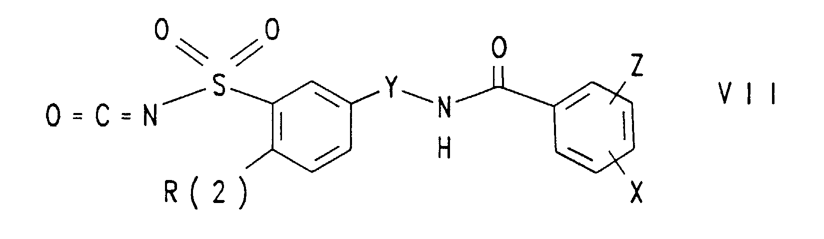 herstellt,
oder
(f) einen Benzolsulfonylthioharnstoff I b durch Umsetzung eines Amins der Formel R(1)-NH₂ mit einem Benzolsulfonylisothiocyanat der Formel VIII 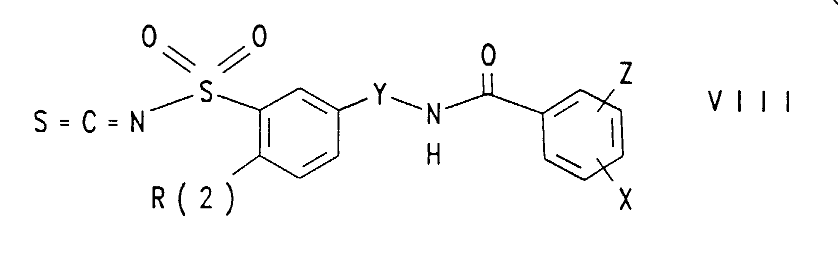 herstellt.

5. Verwendung einer Verbindung I nach einem oder mehreren der Ansprüche 1 bis 3 und/oder eines physiologisch unbedenklichen Salzes davon zum Herstellen eines Medikaments zum Behandeln von Herzrhythmusstörungen.

6. Verwendung einer Verbindung I nach einem oder mehreren der Ansprüche 1 bis 3 und/oder eines physiologisch unbedenklichen Salzes davon zum Herstellen eines Medikaments zur Verhinderung des plötzlichen Herztodes.

7. Verwendung einer Verbindung I nach einem oder mehreren der Ansprüche 1 bis 3 und/oder eines physiologisch unbedenklichen Salzes davon zum Herstellen eines Medikaments zur Behandlung oder Prophylaxe von ischämischen Zuständen des Herzens oder des Infarkts.

8. Verwendung einer Verbindung I nach einem oder mehreren der Ansprüche 1 bis 3 und/oder eines physiologisch unbedenklichen Salzes davon zum Herstellen eines Medikaments zum Cardioprotektion.

9. Verwendung einer Verbindung I nach einem oder mehreren der Ansprüche 1 bis 3 und/oder eines physiologisch unbedenklichen Salzes davon zum Herstellen eines cardioprotektiven Medikaments zur Prophylaxe des Infarkts und der Angina pectoris.

10. Medikament, dadurch gekennzeichnet, daß es eine wirksame Menge einer Verbindung I nach einem oder mehreren der Ansprüche 1 bis 3 und/oder eines physiologisch unbedenklichen Salzes davon enthält.

## Claims

1. A substituted benzenesulfonylurea or -thiourea I 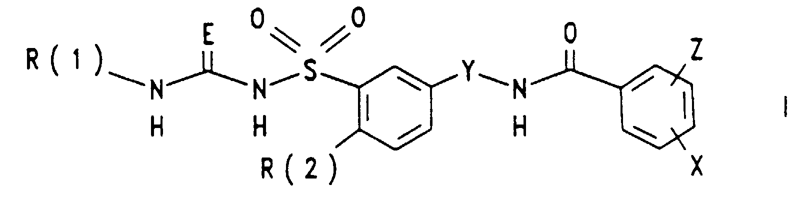 in which:
R(1) is hydrogen, (C₁-C₆) -alkyl, (CH₂)ᵣ-CₚF₂ₚ₊₁, (C₃-C₆)-cycloalkyl, (C₁-C₂) -alkyl-(C₃-C₅)-cycloalkyl or (C₂-C₆)-alkenyl;
r is zero, 1, 2, 3, 4 or 5;
p is 1, 2, 3, 4, 5 or 6;
R(2) is NR (3) R(4),
R(3) and R(4) together are a (CH₂)₂₋₇ chain in which, for a chain length of 4 to 7, one of the CH₂ groups can be replaced by oxygen, sulfur or NR(5), where there must be at least one CH₂ group between the N atom of the NR(3) R(4) and the oxygen, sulfur or NR(5);
or
R(3), R(4) and R(5) independently of one another are
hydrogen, (C₁- C₆)-alkyl, (CH₂)ᵣ-CₚF₂ₚ₊₁, (C₃-C₆)-cycloalkyl, (C₁-C₂)-alkyl-(C₃-C₅)-cycloalkyl or (C₂-C₆)-alkenyl;
r is zero, 1, 2, 3, 4 or 5;
p is 1, 2, 3, 4, 5 or 6;
E is oxygen or sulfur;
Y is a hydrocarbon chain of the formula -[CR(6)₂]ₙ-;
R(6) is hydrogen or (C₁-C₂)-alkyl;
n is 1, 2, 3 or 4;
X is hydrogen, F, Cl, Br, I or (C₁-C₆)-alkyl;
Z is F, C1, Br, I, NO₂, (C₁-C₄)-alkoxy or (C₁-C₄)-alkyl;
or its physiologically acceptable salts.

2. A compound of the formula I as claimed in claim 1, wherein:
R(1) is hydrogen, (C₁-C₄)-alkyl, CₚF₂ₚ₊₁, (C₃-C₅)-cycloalkyl, CH₂-(C₃-C₅)-cycloalkyl or (C₃-C₄)-alkenyl;
p is 1, 2 or 3;
R(2) is NR(3) R(4),
(R3) and R(4) together are a (CH₂)₂₋₇ chain in which, for a chain length of 4 to 7, one of the CH₂ groups can be replaced by oxygen, sulfur or NR(5), where there must be at least one CH₂ group between the N atom of the NR(3) R(4) and the oxygen, sulfur or NR(5);
or
R(3), R(4) and R(5) independently of one another are
hydrogen, (C₁-C₆)-alkyl, (CH₂)ᵣ-CₚF₂ₚ₊₁, (C₃-C₆)-cycloalkyl, (C₁-C₂)-alkyl-(C₃-C₅)-cycloalkyl or (C₂-C₆)-alkenyl;
r is zero, 1, 2, 3, 4 or 5;
p is 1, 2, 3, 4, 5 or 6;
E is oxygen or sulfur;
Y is a hydrocarbon chain of the formula -[CR(6)₂]ₙ-;
R(6) is hydrogen or (C₁-C₂)-alkyl;
n is 1, 2, 3 or 4;
X is hydrogen, F, Cl or (C₁-C₄)-alkyl;
Z is Cl, F, (C₁-C₄)-alkyl or (C₁-C₄)-alkoxy.

3. A compound of the formula I as claimed in claim 1 and/or 2, wherein:
R(1) is hydrogen, (C₁-C₄)-alkyl, (C₃-C₄)-cycloalkyl or (C₃-C₄)-alkenyl;
R(2) is NR(3)R(4),
R(3) and R(4) together are
a (CH₂)₄₋₆ chain in which one CH₂ group can be replaced by oxygen, sulfur or N-R(5), where there must be at least one CH₂ group between the N atom of the NR(3) R(4) and the oxygen, sulfur or NR(5),
or
R(3) and R(4) independently of one another are
CH₃, C₂H₅, n-propyl, isopropyl or cyclopropyl;
R(5) is hydrogen, CH₃ or C₂H₅;
E is oxygen or sulfur,
Y is a hydrocarbon chain of the formula - [CR(6)₂]ₙ- ;
R(6) is hydrogen or methyl;
n is 2 or 3;
X is hydrogen, Cl, For (C₁-C₃)-alkyl;
Z is F, Cl or (C₁-C₃)-alkoxy.

4. A process for the preparation of a compound I as claimed in one or more of claims 1 to 3, which comprises
(a) reacting aromatic sulfonamides of the formula II or their salts of the formula III 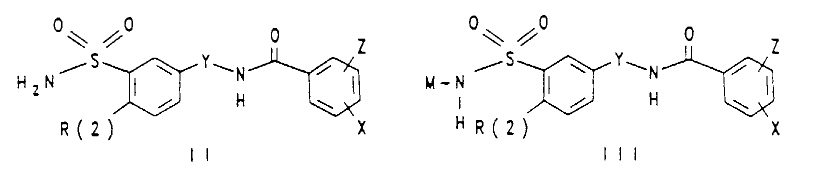 with R(1)-substituted isocyanates of the formula IV
R(1) - N = C = O IV
to give substituted benzenesulfonylureas I a, or
(b) preparing an unsubstituted benzenesulfonylurea I a [R(1) = H] 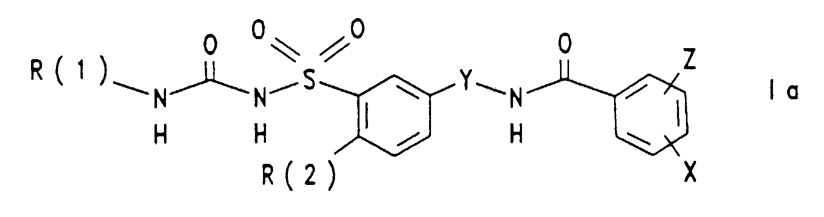 by reactions of an aromatic benzenesulfonamide of the formula II or of its salt III with trialkylsilyl isocyanate or silicon tetraisocyanate and hydrolysis of the primary silicon-substituted benzenesulfonylurea,
or
(c) preparing a benzenesulfonylurea I a 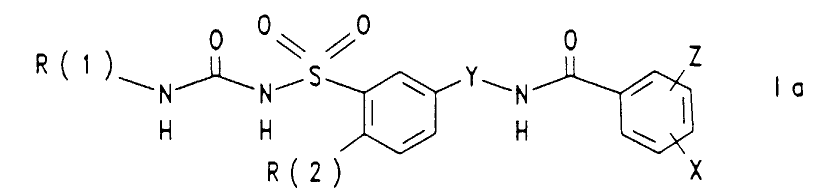 from an aromatic benzenesulfonamide II or its salt III and R(1)-substituted trichloroacetamides of the formula V 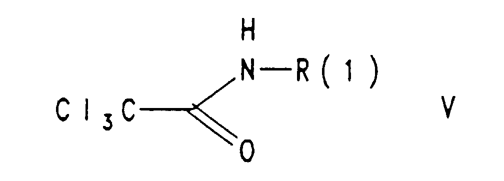 in the presence of a base,
or
(d) preparing a benzenesulfonylthiourea I b 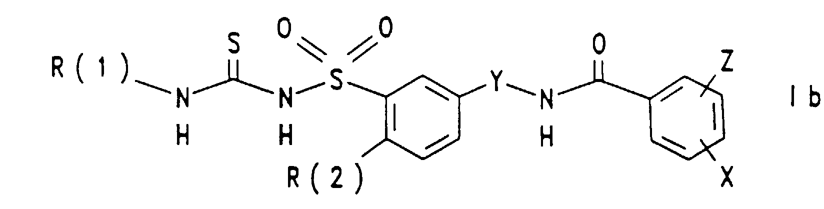 from a benzenesulfonamide II or its salt III and R(1)-substituted thioisocyanates IV
R(1) - N = C = S IV,
or
(e) preparing a benzenesulfonylurea I a by reaction of an amine of the formula R(1)-NH₂ with a benzenesulfonyl isocyanate of the formula VII 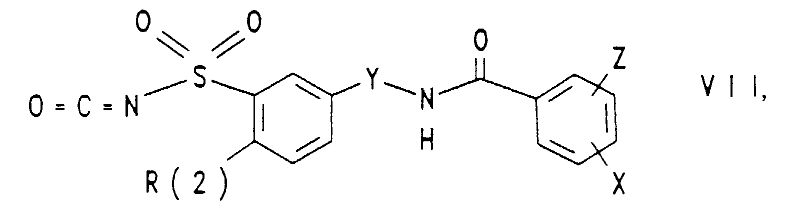 or
(f) preparing a benzenesulfonylthiourea I b by reaction of an amine of the formula R(1)-NH₂ with a benzenesulfonyl isothiocyanate of the formula VIII

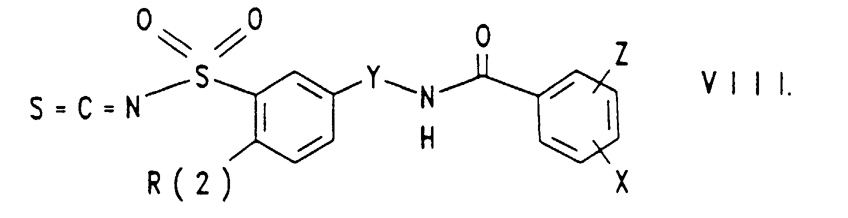

5. The use of a compound I as claimed in one or more of claims 1 to 3 and/or of a physiologically acceptable salt thereof for preparing a medicament for treating cardiac arrhythmias.

6. The use of a compound I as claimed in one or more of claims 1 to 3 and/or of a physiologically acceptable salt thereof for preparing a medicament for the prevention of sudden heart death.

7. The use of a compound I as claimed in one or more of claims 1 to 3 and/or of a physiologically acceptable salt thereof for preparing a medicament for the treatment or prophylaxis of ischemic conditions of the heart or of infarct.

8. The use of a compound I as claimed in one or more of claims 1 to 3 and/or of a physiologically acceptable salt thereof for preparing a medicament for cardioprotection.

9. The use of a compound I as claimed in one or more of claims 1 to 3 and/or of a physiologically acceptable salt thereof for preparing a cardioprotective medicament for prophylaxis of infarct and of angina pectoris.

10. A medicament which contains an effective amount of a compound I as claimed in one or more of claims 1 to 3 and/or of a physiologically acceptable salt thereof.

## Revendications

1. Phénylsulfonyl-urées et -thiourées substituées de formule (I) 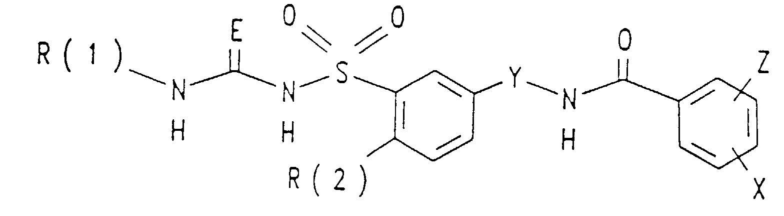 dans laquelle
R (1) représente un atome d'hydrogène, (C₁-C₆)alkyl, (CH₂)ᵣ-CₚF₂ₚ₊₁, (C₃-C₆)-Cycloalkyle, (C₁-C₂)-Alkyl-(C₃-C₅) cycloalkyle ou (C₂-C₆)-alcényle ;
r réprésente 0,1,2,3,4 ou 5;
p représente 1,2,3,4,5 ou 6;
R (2) représente NR(3)R(4), où
R(3) et R(4) représentent ensemble
une chaîne (CH₂)₂₋₇ dans laquelle, lorsque ladite chaîne comprend de quatre à sept atomes de carbone l'un des groupes CH₂ peut être remplacé par un atome d'oxygène, un atome de soufre ou NR(5), étant entendu qu'au moins un groupe CH₂ doit être présent entre l'atome d'azote de NR(3)R(4) et l'atome d'oxygène, l'atome de soufre ou NR(5);
ou
R(3), R(4), R(5) indépendamment les uns des autres représentent
un atome d'hydrogène, (C₁-C₆)-alkyle, (CH₂)ᵣ-CₚF₂ₚ₊₁, (C₃-C₆)-cycloalkyle,(C₁-C₂)-alkyl-(C₃-C₅)-cycloalkyle ou (C₂-C₆)-alcényle;
r représente 0, 1, 2, 3, 4 ou 5;
p représente 1, 2, 3, 4, 5 ou 6;
E représente un atome d'oxygène ou un atome de soufre;
Y représente une chaîne hydrocarbonée de formule :
-[(CR(6)₂]ₙ-;
R(6) représentant un atome d'hydrogène ou (C₁-C₂)-alkyle ;
n représente 1, 2, 3 ou 4
X représente un atome d'hydrogène ou F, Cl, Br, I ou (C₁-C₈)-alkyle ;
Z représente F, Cl, Br, I, NO₂, (C₁-C₄)-alkoxy ou (C₁-C₄)-alkyle ; et leurs sels physiologiquement acceptables.

2. Composé de formule I selon la revendication 1, caractérisé en ce que :
R1 représente un atome d'hydrogène, (C₁-C₄)-alkyle, CₚF₂ₚ₊₁, (C₃-C₅)-cycloalkyle, CH₂-(C₃₋C₅)-cycloalkyle ou (C₃-C₄)-alcényle;
p représente 1, 2 ou 3;
R(2) représente NR(3)R(4),
R(3)R(4) représentent ensemble une chaîne (CH₂)₂₋₇, dans laquelle lorsque ladite chaîne contient de 4 à 7 atomes de carbone, un groupe CH₂ peut être remplacé par un atome d'oxygène, un atome de soufre ou NR(5); étant entendu qu'au moins un groupe CH₂ doit être présent entre l'atome d'azote de NR(3)R(4) et l'atome d'oxygène, l'atome de soufre ou NR(5) ;
ou
R(3), R(4), R(5) représentent indépendamment un atome d'hydrogène, (C₁-C₆)-alkyle, (CH₂)ᵣ-CₚF₂ₚ₊₁, (C₃-C₆)-cycloalkyle, (C₁-C₂)-alkyl-(C₃-C₅)-cycloalkyle ou (C₂-C₆)-alcényle;
r représente 0, 1, 2, 3, 4 ou 5
p représente 1, 2, 3, 4, 5 ou 6
E représente un atome d'oxygène ou un atome de soufre;
Y représente une chaîne hydrocarbonée de formule
-[CR(6)₂]ₙ-,
R(6) représente un atome d'hydrogène ou (C₁-C₂)alkyle ;
n représente 1, 2, 3 ou 4,
X représente un atome d'hydrogène, F, Cl ou (C₁-C₂)-alkyle;
Z représente Cl, F, (C₁-C₄)-alkyle ou (C₁-C₄)-alkoxy.

3. Composé de formule (I) selon la revendication 1 ou la revendication 2, caractérisé en ce que
R(1) représente un atome d'hydrogène, (C₁-C₄)-alkyle, (C₃-C₄)-cycloalkyle ou (C₃-C₄)-alcényle;
R(2) représente NR(3)R(4),
où R(3) et R(4) représentent ensemble
une chaîne (CH₂)₄₋₆, dans laquelle un groupe -CH₂- peut être remplacé par un atome d'oxygène, un atome de soufre ou NR(5), étant entendu qu'au moins un groupe -CH₂- doit être présent entre l'atome d'azote de NR(3)R(4) et l'atome d'oxygène, l'atome de soufre ou NR(5)
ou bien
R(3) et R(4) représentent indépendamment CH₃, C₂H₅, n-propyle, isopropyle ou cyclopropyle;
R(5) représente un atome d'hydrogène, CH₃ ou C₂H₅;
E représente un atome d'oxygène, ou un atome de soufre;
Y représente une chaîne hydrocarbonée de formule
-[CR(6)₂]ₙ-;
R(6) représente un atome d'hydrogène ou un groupe méthyle;
n représente 2 ou 3;
X représente un atome d'hydrogène, Cl, F ou un groupe (C₁-C₃)-alkyle;
Z représente F, Cl ou (C₁-C₃)-alkoxy.

4. Procédé pour la préparation d'un composé de formule I selon une ou plusieurs des revendications 1 à 3, caractérisé en ce que :
a) on fait réagir un sulfonamide aromatique de formule Il ou son sel de formule III 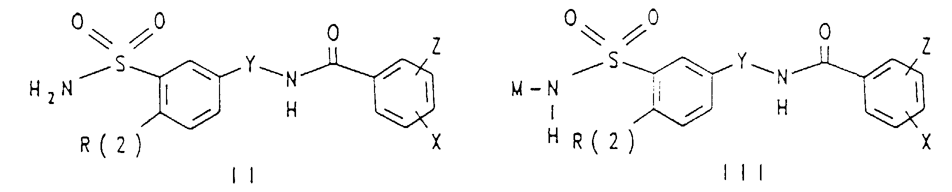 avec un isocyanate substitué par R(1) de formule IV
R(1) - N = C = O IV
pour la préparation d'une phénylsulfonylurée substituée de formule I; ou bien
b) on prépare une phénylsulfonylurée non-substituée de formule I a [R(1) = H] 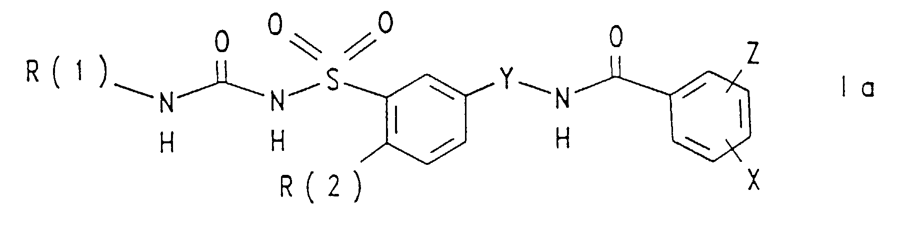 par réaction par réaction d'un phénylsulfonamide aromatique de formule II ou de son sel III avec un trialkylsilylisocyanate ou bien un tétraisocyanate de silicium, et hydrolyse de la phénylsulfonylurée primaire substituée par du silicium,
ou bien
c) on produit une phénylsulfonylurée de formule I a 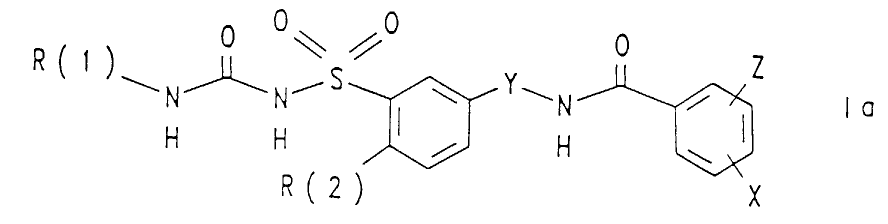 à partir d'un phénylsulfonamide aromatique Il ou de son sel III et d'un trichloracétamide de formule V 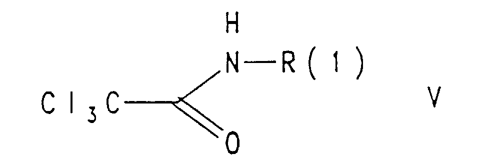 en présence d'une base,
ou bien
(d) on obtient une phénylsulfonylthiourée de formule I b 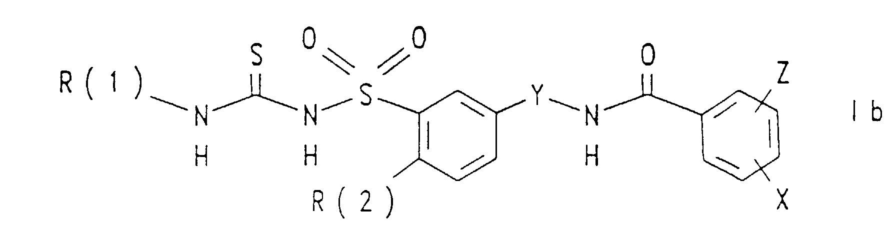 à partir d'un phénylsulfonamide Il ou de son sel III et d'un thioisocyanate IV substitué par R(1)
R(1) - N = C = S IV
ou bien
(e) on prépare une phénylsulfonylurée de formule I a par réaction d'une amine de formule R(1)-NH₂ avec un phénylsulfonylisocyanate de formule VII 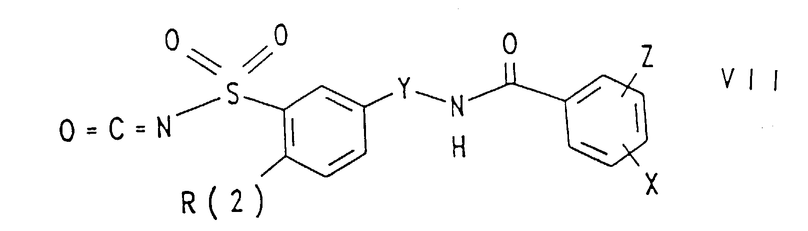 ou bien
(f) on prépare une phénylsulfonylthiourée de formule I b par réaction d'une amine de formule R(1)-NH₂ avec un phénylsulfonylisothiocyanate de formule VIII

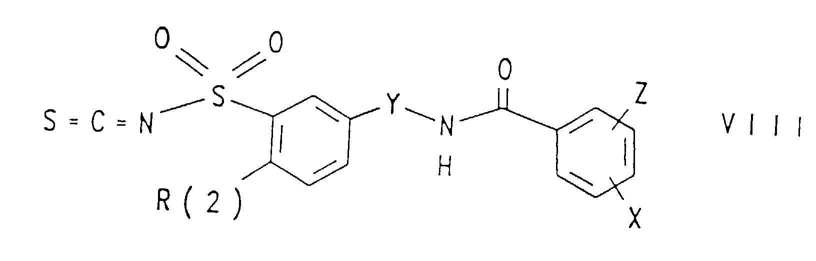

5. Utilisation d'un composé de formule I selon l'une ou plusieurs des revendications 1 à 3 et/ou de l'un de ses sels physiologiquement acceptables pour la préparation d'un médicament pour le traitement de troubles du rythme cardiaque.

6. Utilisation d'un composé de formule I selon l'une ou plusieurs des revendications 1 à 3, et/ou de l'un de ses sels physiologiquement acceptables pour la préparation d'un médicament destiné à prévenir la mort-subite.

7. Utilisation d'un composé de formule I selon une ou plusieurs des revendications 1 à 3 et/ou d'un de ses sels physiologiquement acceptables pour la préparation d'un médicament pour le traitement de la cardioischémie ou de l'infarctus.

8. Utilisation d'un composé de formule I selon une ou plusieurs des revendications 1 à 3 et/ ou de l'un de ses sels physiologiquement acceptables, pour la préparation d'un médicament cardio-protecteur.

9. Utilisation d'un composé de formule I, selon une ou plusieurs revendications 1 à 3 et/ou de l'un de ses sels physiologiquement acceptables pour la préparation d'un médicament cardio-protecteur dans le traitement prophylactique de l'infarctus et de l'angine de poitrine.

10. Médicament caractérisé en ce qu'il contient une quantité suffisante pour être actif d'un ou plusieurs composé de formule I selon l'une des revendications 1 à 3 et/ou d'un de ses sels physiologiquement acceptables.
